# EUROPÄISCHE PATENTSCHRIFT

(11) **EP 3 747 018 B1**
(45) Veröffentlichungstag und Bekanntmachung des Hinweises auf die Patenterteilung: **29.04.2026**
(21) Anmeldenummer: 19702550.5
(22) Anmeldetag: 25.01.2019
(51) Int. Cl.: G16H 40/60

(54) **ÜBERWACHUNG VON BEDIENAKTIONEN FÜR EIN DIALYSEGERÄT**
MONITORING OPERATING ACTIONS FOR A DIALYSIS DEVICE
SURVEILLANCE DE MANIPULATION POUR UN APPAREIL DE DIALYSE

(30) Priorität: 29.01.2018 DE 102018101893
(43) Veröffentlichungstag der Anmeldung: 09.12.2020
(73) Patentinhaber: Fresenius Medical Care Deutschland GmbH, 61352 Bad Homburg (DE)
(72) Erfinder: SCHROERS, Alexander, 60389 Frankfurt (DE); NADIG, Vanessa, 52062 Aachen (DE)
(74) Vertreter: Schwarz, Claudia
(86) Internationale Anmeldenummer: PCT/EP2019/051859
(87) Internationale Veröffentlichungsnummer: WO 2019/145477

(56) Entgegenhaltungen:
- US-A- 5 472 614
- US-A1- 2009 106 605
- US-A1- 2016 341 564
- US-A1- 2017 065 757

## Beschreibung

Die vorliegende Erfindung betrifft die Überwachung der Gerätebedienung von Dialysegeräten oder anderen medizintechnischen Geräten. Sie bezieht sich insbesondere auf eine Bedienungsprüfeinheitseinheit, auf ein medizinisches Gerät mit einer solchen Bedienungsprüfeinheitseinheit und auf ein Verfahren zum anwenderspezifischen Überwachen von Bedienaktionen beim Betrieb des Dialysegerätes.

In der klinischen Praxis ist das fehlerfreie Funktionieren des medizintechnischen Gerätes unabdingbar. Das Gerät besteht aus einer Vielzahl von elektronischen und/oder technischen Einheiten, die ebenfalls fehlerfrei funktionieren müssen. Um dies sicherzustellen, sind in dem Gerät eine Vielzahl von Meldern (z.B. Sensoren) verbaut, die einen Zustand des Gerätes mit seinen Einheiten erfassen und diesen bedarfsweise und insbesondere im Fehlerfall mittels einer Meldung über die Benutzerschnittstelle dem Anwender melden. Ist z.B. eine Pumpe oder ein anderes Bauteil des Gerätes ausgefallen oder nicht mehr funktionsfähig, wird dies über eine entsprechende Sensorik als ein Fehlerzustand des Gerätes erfasst. Ein Fehlerzustand des Gerätes kann somit schnell erfasst und gemeldet werden.

Fehler an dem Gerät können allerdings nicht nur durch fehlerhafte technische Geräte-Komponenten entstehen, sondern auch durch eine Fehlbedienung seitens des Anwenders. Bei Hämodialyse-Behandlungen muss beispielsweise beim Einlegen der Heparinspritze zur Antikoagulierung des Blutes im extrakorporalen Blutkreislauf darauf geachtet werden, dass der Drückstempel der motorisch betriebenen Spritzenpumpe mit dem Stößel der Spritze kraftschlüssig verbunden ist. Ist dies nicht der Fall, kann aufgrund der minimalen Pumprate dieser speziellen Pumpe eine lange Zeit vergehen, in der kein Heparin in das extrakorporale Blut gefördert wird. Dies kann zur Folge haben, dass das Blut im extrakorporalen Blutkreislauf gerinnt (Clotting), wodurch der Filter verstopfen kann, oder im Extremfall sogar geronnenes Blut zurück in das vaskuläre System des Patienten gefördert werden kann (Gefahr einer Embolie).

Ein derartiger Fehler kann mitunter lebensbedrohlich für den Patienten sein.

Die Druckschrift US 2017/065757 A1 beschreibt eine Sprachschnittstelle für ein Dialysegerät mit einem Lautsprecher sowie einem Mikrofon und einer Spracherkennungseinheit. Wird festgestellt, dass eine erforderliche Handlung nicht abgeschlossen ist, so können akustische Anweisungen hierzu ausgegeben werden. Wird festgestellt, dass eine Handlung abgeschlossen ist, so können Anweisungen für eine weitere, nachfolgende Handlung ausgegeben werden.

Es besteht im Stand der Technik ein Bedarf, Bedienaktionen von Anwendern des Gerätes auf Fehlerfreiheit zu überwachen und proaktive automatische Hilfsmaßnahmen auslösen zu können.

Ausgehend von den bekannten Systemen als Stand der Technik hat sich die vorliegende Erfindung deshalb zur Aufgabe gestellt, die Sicherheit des Gerätebetriebs zu verbessern und die Anzahl von Fehlbedienungen und insbesondere die Ausfallzeit des Gerätes verursacht durch Fehlbedienungen zu reduzieren. Ferner soll ein effizienteres Fehlerhandling für den Gerätebetrieb bereitgestellt werden.

Diese Aufgabe wird erfindungsgemäß durch eine Bedienungsprüfeinheitseinheit, ein Dialysegeräte und ein Verfahren gemäß den beiliegenden, einander nebengeordneten Patentansprüchen gelöst. Weitere vorteilhafte Ausführungsformen sind Gegenstand der abhängigen Ansprüche.

Im Folgenden wird die Erfindung anhand der vorrichtungsgemäßen Aufgabenlösung, und somit anhand der Bedienungsprüfeinheitseinheit beschrieben. Dabei erwähnte Merkmale, Vorteile oder alternative Ausführungsformen sind ebenso auch auf die anderen beanspruchten Gegenstände zu übertragen und umgekehrt. Mit anderen Worten können auch die verfahrensbasierten Ansprüche mit den Merkmalen weitergebildet sein, die in Zusammenhang mit der Bedienungsprüfeinheitseinheit beschrieben und/oder beansprucht sind und umgekehrt. Die entsprechenden funktionalen Merkmale des Verfahrens werden dabei durch entsprechende gegenständliche Module, insbesondere durch elektronische Hardware-Module oder Mikroprozessor-Module, des Systems bzw. des Produktes ausgebildet und umgekehrt. So kann z.B. das Erfassen einer Anwenderidentität durch eine Identifikationseinheit ausgeführt werden, die z.B. eine Kamera umfasst und ein optisches Identifikationsverfahren ausführt.

Ein ersten Aspekt betrifft eine Bedienungsprüfeinheitseinheit zur Überprüfung von Bedienaktionen eines Anwenders auf Fehlerfreiheit und zur anwenderspezifischen, proaktiven Steuerung eines Dialysegerätes. Die Bedienungsprüfeinheitseinheit umfasst dazu:
- Eine Identifikationseinheit, die dazu ausgebildet ist, eine Anwenderidentität eines Anwenders zu erfassen; dies kann insbesondere während oder vor einer Bedienaktion an dem Dialysegeräte ausgeführt werden;
- Eine Sensoreinheit, die ausgebildet ist, eine Bedienaktion des identifizierten Anwenders als IST-Bediendatensatz zu erfassen;
- Eine Verarbeitungseinheit, die dazu bestimmt ist, den erfassten IST-Bediendatensatz mit einem in einem Speicher gespeicherten SOLL-Bediendatensatz auf Übereinstimmung zu vergleichen, um bei Abweichung eine anwenderspezifische Meldung zu erzeugen.

Ein wesentlicher Gedanke der Erfindung ist darin zu sehen, dass die Gerätebedienaktionen eines spezifischen Anwenders zum Betreiben des Gerätes individuell erfasst und ausgewertet werden, um eine an die jeweilige Bedienung des Anwenders und somit eine anwenderspezifische Steuerung des Gerätes mit proaktiv generierten Bedienhinweisen und Hilfen zum verbesserten und insbesondere fehlerfreien Bedienen des Gerätes zu initiieren. Ein erster Anwender kann somit andere Bedienhinweise erhalten als ein zweiter Anwender. Durch die erfindungsgemäße Überwachung der Bedienaktionen, kann die jeweilige anwenderspezifische Meldung sogar vor dem intendierten Gerätebedienungsschritt ausgeführt werden, um Fehlbedienungen des Anwenders, die in der Vergangenheit erfasst worden sind, zukünftig zu vermeiden.

Im oben erwähnten Beispiel zum Einlegen der Heparinpumpe kann der geschilderte Fehler gemäß einer bevorzugten Ausführungsform der Erfindung beispielsweise durch die Überwachung des Motorstroms der Spritzenpumpe bemerkt werden, der bei einer kraftschlüssig eingelegten Heparinspritze aufgrund des dann vorhandenen Gegendrucks einen anderen charakteristischen Verlauf annimmt als bei einem Leerlauf des Druckstempels.

In einer Ausführungsform umfasst die Bedienungsprüfeinheit eine Auswerteeinheit, die dazu ausgebildet ist, auszuwerten, ob, wie oft, in welchem Zeitraum und/oder in welchem Betriebsmodus die (jeweils aktuelle) Bedienaktion erfasst worden ist und ein Auswertungsergebnis bereitzustellen. Das Auswerteergebnis hat damit vorteilhafterweise eine höhere Aussagekraft und kann spezifischer auf die Bediensituation abgestellt sein, um die proaktive Gerätesteuerung mit der Ausgabe von zielführenden Hinweisen zu verbessern.

In einer weiteren Ausführungsform umfasst die Bedienungsprüfeinheit eine Ausgabeeinheit, die dazu ausgebildet ist, die anwenderspezifische Meldung, den erfassten IST-Bediendatensatz, den SOLL-Bediendatensatz und/oder ein Auswerteergebnis auszugeben. Dabei kann es sich z.B. um ein Bedienfeld des Gerätes handeln. Damit kann der Anwender dediziert mit speziell für ihn hilfreichen Bedienhinweisen ausgestattet werden.

In einem weiteren Aspekt bezieht sich die Erfindung auf ein Dialysegerät, mit einer Bedienungsprüfeinheit wie vorstehend beschrieben.

Ein weiterer Aspekt bezieht sich auf ein Verfahren zum anwenderspezifischen Überwachen von Bedienaktionen beim Betrieb eines Dialysegerätes und zur anwenderspezifischen Steuerung des Gerätes. Dabei wird während eines Betriebs des Dialysegerätes (umfassend ein Aufrüsten des Gerätes bzw. eine Inbetriebnahme, Hochfahren, Normalbetrieb, Servicebetrieb etc.) zunächst eine Anwenderidentität und daraufhin eine Bedienaktion des identifizierten Anwenders als IST-Bediendatensatz erfasst. Der erfasste IST-Bediendatensatz wird dann einer konfigurierbaren Datenverarbeitung zugeführt, insbesondere um diesen mit einem gespeicherten SOLL-Bediendatensatz auf Übereinstimmung zu vergleichen, um bei Abweichung eine anwenderspezifische Meldung zu erzeugen.

In einer weiteren Ausführungsform erfolgt das Erfassen der Bedienaktion in einer ersten Betriebsphase und ein Ausgeben der erzeugten anwenderspezifischen Meldung in einer zweiten Betriebsphase. "Betriebsphase" meint hier eine Bedienphase des Anwenders. Diese kann sich insbesondere auf eine bestimmte Prozedur auf dem Gerät oder auf eine Abfolge von konsekutiven Bedienaktionen beziehen. Dies hat z.B. den Vorteil, dass aufgrund von vorhergehenden Fehlerüberwachungen von Bedienaktionen für dieselbe Prozedur (die eine Abfolge von Bedienaktionen umfassen kann) und für den identischen Anwender festgestellt worden ist, dass dem Anwender hier Fehler unterlaufen sind, so kann diese Information zur modifizierten Gerätesteuerung genutzt werden. Insbesondere kann das System proaktiv die Ausgabe eines Benutzerhinweises triggern, um den Benutzer über die korrekte Ausführung der anstehenden Bedienaktion zu informieren und gegebenenfalls weiterhin darüber zu informieren, dass ihm bisher bei der intendierten Bedienaktion immer Fehler unterlaufen hin. Insbesondere kann ihm auch der konkrete Fehler angezeigt werden, zusammen mit der korrekten Ausführung. Zum Beispiel kann sich die Bedienaktion auf das Auswechseln / den Einbau eines Filters beziehen. Wenn in einer zurückliegenden, früheren Betriebsphase erfasst worden ist, dass der Anwender den Filter nicht gedreht hat, was aber notwendig ist, so kann ihm diese Information vor der intendierten Gerätebetriebsmaßnahme zur Kenntnis gebracht werden, um ihn proaktive auf die korrekte Ausführung der Bedienaktion bereits im Vorfeld zu informieren. Damit kann das Risiko für anwenderspezifische Fehler bereits im Vorfeld deutlich reduziert werden.

In einer Ausführungsform wird somit die anwenderspezifische Meldung ausgegeben bevor die Bedienaktion ausführt wird, wobei die anwenderspezifische Meldung aus einer vorhergehenden Betriebsphase erfasst worden ist.

In einer Ausführungsform wird somit die erzeugte anwenderspezifische Meldung bereits vor der jeweiligen meldungsverursachenden Bedienaktion ausgegeben, wenn in einer vorhergehenden Betriebsphase des identifizierten Anwenders für die jeweilige Bedienaktion eine anwenderspezifische Meldung erzeugt worden ist. Die Bedienaktion kann in eine Abfolge von aufeinander folgenden (vordefinierten aufeinander abgestimmten und/oder aufeinander aufbauenden) Bedienaktionen eingebettet sein. Die Meldung kann eine fehlerhafte Bedienung des Gerätes indizieren. Die anwenderspezifische Meldung kann somit ausgegeben werden, unmittelbar bevor eine weitere, möglicherweise fehlerindizierende Bedienaktion ausgeführt werden soll. Die Fehlermeldung ist in diesem Fall ein Warnhinweis und soll ein Korrektiv für das Verhalten des Anwenders darstellen.

In einer weiteren Ausführungsform werden mit dem Erfassen der Bedienaktion weitere Metadaten, insbesondere ein Zeitstempel, erfasst. Damit kann eine umfangreichere Auswertung der Bedienaktionen ausgeführt und ein detailreicheres Auswertungsergebnis bereitgestellt werden.

In einer weiteren Ausführungsform wird die Anwenderidentität erfasst, indem ein Identifikationsprozess ausgeführt. Der Identifikationsprozess kann auf einem optischen Verfahren (Kamera, Augenscan) und/oder einem biometrischen Verfahren (Fingerabdruck) und/oder auf einer Eingabe von Schlüsseldaten (Passwort, Benutzername) und/oder auf einem Einlesen von Daten eines mobilen Datenträgers (Smartcard, Pager ...) basieren. Die vorstehend aufgelisteten Möglichkeiten können auch kumulativ zum Einsatz kommen, Damit kann die Sicherheit der Anwenderidentifikation erhöht werden.

In einer weiteren, Ausführungsform wird die anwenderspezifische Meldung auf einem Monitor des Dialysegerätes, insbesondere auf einem Touchscreen-Monitor ausgegeben. Dies hat den Vorteil, dass der Anwender ohnehin seine Aufmerksamkeit auf dem Bedienmonitor lenkt und nicht von einem weiteren Gerät abgelenkt wird. Alternativ kann die Meldung auch an andere, externe Instanzen zum Zwecke des Lehrens und Schulens des Anwenders weitergeleitet werden. Auch kann die Meldung und das Auswertungsergebnis an einen zentralen Server weitergeleitet werden, z.B. um statistische Auswertungen auszuführen.

In einer weiteren Ausführungsform erfordert die anwenderspezifische Meldung eine Quittierung seitens des Anwenders. Damit kann sichergestellt werden, dass der Anwender die Meldung auch bestätigen muss, bevor das Gerät weiter bedient werden kann. Umgekehrt kann damit das Risiko verringert werden, dass die Meldung, die ihm als Hinweis dienen soll, übersehen wird. Die Quittierung wird technisch durch das Erfassen eines Quittierungssignals implementiert. Die Meldung kann eine fehlerhafte Bedienung indizieren. Die Meldung kann Bedienhinweise (als Hilfe) umfassen.

In einer weiteren, Ausführungsform ist ein Ausgeben der anwenderspezifischen Meldung aktivierbar und de-aktivierbar. Damit kann auf der einen Seite das Erfassen der Bedienaktionen fortgeführt und fortgeführt gespeichert werden und auf der anderen Seite ist das Ausgeben der Meldung quasi "abschaltbar". Dies macht das Verfahren flexibel einsetzbar. So kann z.B. in eilbedürftigen Notsituationen die Ausgabe von Hinweisen und Warnungen abgestellt werden, so dass auch keine Quittierungen eingegeben werden müssen, wodurch der Betrieb beschleunigt werden kann. Die Meldung kann auch automatisch nach Eintreten eines vordefinierbaren Ereignisses ausgelöst werden. So können. Regeln konfiguriert und insbesondere lokal in dem Speicher des Gerätes hinterlegt werden, die z.B. festlegen, dass eine Meldung dann erfolgt, wenn dreimal in Folge eine Fehlbedienung des Anwenders für dieselbe Prozedur erfasst worden ist oder falls es sich um eine sicherheitskritische Prozedur handelt.

In einer weiteren Ausführungsform werden alle erfassten Bedienaktionen von allen Anwendern aggregiert und für jeden Anwender aufgeschlüsselt gespeichert. Dies kann lokal an dem Gerät oder extern auf einem zentralen Server ausgeführt werden. Der zentrale Server oder eine zentrale Einheit steht mittels Datenübertragung mit dem Gerät in Verbindung. Alternativ können die verarbeiteten Daten, insbesondere das Auswertungsergebnis oder die Meldungen auf der zentralen Einheit ausgegeben werden. Damit kann das System auch als Schulungssystem zur Ausbildung eingesetzt werden.

In einer weiteren Ausführungsform wird der SOLL- Bediendatensatz in Abhängigkeit von der erfassten Anwenderidentität und/oder von einem Betriebsmodus anwenderindividuell konfiguriert. Dies steigert die Flexibilität des Verfahrens.

Im Folgenden werden die im Rahmen dieser Anmeldung verwendeten Begrifflichkeiten definiert.

Die Bedienungsprüfeinheitseinheit ist eine elektronische Komponente. Sie kann in Hardware (als elektronische Schaltungseinheit, z.B. mittels eines ASIC, FPGA oder DSP etc.) und/oder Software oder Firmware ausgebildet sein und dient zur Überwachung von Bedienaktionen, die am Gerät ausgeführt werden und soll insbesondere eine korrekte Bedienung sicherstellen. Des Weiteren dient die Bedienungsprüfeinheitseinheit zur Steuerung des Gerätes über die Erzeugung und Ausgabe von Meldungen oder eines Prüfergebnisses oder Verarbeitungsergebnisses, die/das spezifisch an die zuvor erfassten Bedienaktionen angepasst sind. Die Bedienungsprüfeinheitseinheit ist vorzugsweise auf dem Gerät implementiert. Die Bedienungsprüfeinheitseinheit kann Schnittstellen zu externen Einheiten (z.B. zentraler Server) umfassen.

Im Folgenden wird die Erfindung für ein Dialysegerät als Beispiel eines medizintechnischen Gerätes beschrieben, z.B. eines Hämodialysegerätes oder eines Peritonealdialysegerätes. Für den Fachmann liegt es jedoch auf der Hand, dass die Erfindung ebenso auf andere medizintechnische, computergesteuerte Geräte oder (Fluidmanagement-) Maschinen oder Blutbehandlungsgeräte angewendet oder übertragen werden kann, die von einem Anwender über Bedienaktionen, die am Geräte auszuführen sind, bedient werden.

Der Betrieb des Gerätes bezieht sich auf alle Betriebszustände, in denen das Gerät aktiv ist oder aktiviert wird, wie z.B. beim Hochfahren, Aufrüsten, bei einem normalen Betrieb (z.B. während der Dialysebehandlung), einem Servicebetrieb, eine Wartung, ein Shut-Down. Die vorstehend genannte Aufzählung ist nicht abschießend. Der Betrieb kann sich auch auf einen Teilbetrieb nur eines Gerätebauteils beziehen, wie z.B. ein Desinfizieren des extrakorporalen Blutkreislaufs, Füllen und Entlüften des extrakorporalen Blutkreislaufs, Reaktion auf Alarmmeldungen, Ende der Behandlung mit Blutrückgabe an den Patienten, Entleeren des extrakorporalen Blutkreislaufs, Aufrufen bestimmter Menüpunkte auf der Benutzeroberfläche (z.B. GUI).

Die Anwenderidentität ist ein elektronischer Datensatz, der die Identität des (aktuellen) Anwenders am Gerät eindeutig kennzeichnet. Sie kann bei einer Gerätebedienung wechseln und sich in einem ersten Zeitraum von einem zweiten Zeitraum unterscheiden. Die Anwenderidentität kann ein den Anwender eindeutig identifizierender Code sein, wie z.B. eine Personalnummer, die über eine Datenschnittstelle eingelesen wird. Die Anwenderidentität kann durch unterschiedliche technische Maßnahmen erfasst werden, die entweder auf unterschiedlichen Sensoren basieren, die Identifikationsdaten erfassen oder - wie vorstehend bereits beschrieben - durch Einlesen von Identifikationsdaten über eine Schnittstelle (z.B. einem mobilen Datenträger, wie einer Smartcard, einer Smartwatch, einem Pager) oder durch Einlesen eines NFC-Codes, den der Anwender auf einem Datenträger mit sich trägt). Als Sensoren können in einer bevorzugten Ausführungsform der Erfindung beispielsweise visuelle Sensoren zum Einsatz kommen, beispielsweise eine Kamera (CCD-Kamera). Die Anwenderidentität kann mittels biometrischer Verfahren (z.B. mit einem optischen Irisscan und/oder mit der Erfassung eines Fingerabdrucks auf der Benutzeroberfläche des Gerätes) erfasst werden. Ebenso kann dazu ein Authentifizierungsverfahren angewendet werden, wie z.B. im einfachsten Fall die Eingabe eines Benutzernamens und einer die Person identifizierenden geheimen Codes (z.B. Passwort). Die vorstehenden genannten Identifikationsmöglichkeiten können auch kombiniert zur Anwendung kommen, damit kann die Sicherheit der Bedienprüfeinheit verbessert werden.

Bei dem IST- und dem SOLL-Bediendatensatz handelt es sich um elektronische Datentupel. In einer Variante der Erfindung umfassen der IST- und SOLL-Bediendatensatz mehrere Parameter, um mehrere Sensorwerte ein einer vordefinierten Abfolge zu repräsentieren. Die Sensorwerte können eine Abfolge von Bedienaktionen des Anwenders am Gerät kennzeichnen (wie z.B. zunächst Starten des Desinfektionsprozesses auf der Benutzeroberfläche, anschließendes Öffnen der Türe, Abtrennen des Schlauches, ...). Die Bedienaktionen können mit unterschiedlichen Sensoren (unterschiedliche ausgerichtete optischen Sensoren, Schalter, Näherungssensoren etc.) erfasst werden. Der SOLL-Bediendatensatz kann je nach Anwendungsfall konfiguriert werden und/oder von einem zentralen Administrationsknoten eingelesen werden. Damit ist es möglich, die SOLL-Vorgaben leicht und einfach zu aktualisieren oder an den jeweiligen Kontext anzupassen.

Die Meldung ist eine elektronische Nachricht, die anwenderspezifisch ist und die vom Anwender am Gerät ausgeführten Bedienaktionen auf Korrektheit kennzeichnet. Auf einer abstrakten Ebene wird zwischen Fehlermeldungen und Korrektheitsmeldungen unterschieden. Die Fehlermeldungen können noch weitere Hinweise umfassen. Die Meldung kann unmittelbar lokal an dem Gerät ausgegeben werden (z.B. auf dem GUI) und/oder an eine externe Instanz (z.B. Server) über eine Datenverbindung übermittelt werden, um z.B. weitere statistische Auswertungen auszuführen. Am Gerät kann eingestellt werden, dass am Gerät nur Fehlermeldungen ausgegeben werden. Die Meldung kann Metadaten umfassen, die im Kontext der Bedienaktion relevant sind, umfassend gerätebezogenen Metadaten (Gerätezustand, Art des bedienten Betriebsmittels, Betriebszeitpunkt, Betriebsmodus etc.) und bedienbezogene Metadaten (Rolle des Anwenders (Patient, Arzt, Schwester), Häufigkeit der Fehlbedienung, Bedienphase (Notfall oder Normalbetrieb)). Die Meldung basiert somit auf einer Verarbeitung auf dem Gerät. Die Meldung umfasst somit ein Prüfergebnis (als Ergebnis des Vergleichs zwischen SOLL- und IST-Bediendatensatz) und/oder ein Verarbeitungsergebnis. Die Meldung kann somit als Bedienhilfe dienen.

Die Fehlermeldungen können nach einem vorkonfigurierbaren Schema klassifiziert werden, etwa nach Priorität bzw. Schweregrad. Hierbei kann z.B. unterschieden werden in kritische und unkritische Fehler. Unkritische Fehler haben keinen oder nur einen geringen Einfluss auf den Patienten (bspw. unsichere, da langwierige Eingaben am Touchscreen). Kritische Fehler sind Fehler, die potentiell einen bedeutenden Einfluss auf die Behandlung des Patienten haben. Treten solche kritischen Fehler bei einem bestimmten Anwender gehäuft auf, kann dies gemäß einer bevorzugten Ausführungsform der Erfindung automatisch detektiert werden und beispielsweise einer übergeordneten Instanz (Vorgesetzter, Klinikleitung) per elektronischer Nachricht vom Gerät (bzw. über eine in Datenaustausch befindliche Auswerteeinheit, die sich nicht notwendigerweise im medizintechnischen Gerät befinden muss) gemeldet werden. Eine derartige Nachricht kann beispielsweise zum Anlass genommen werden, dem bestimmten Anwender eine gesonderte Schulung zukommen zu lassen, um solche kritischen Bedienfehler künftig zu vermeiden. In einer Erweiterung der Erfindung kann hierbei eine stufenweise Eskalierung der Maßnahmen in Abhängigkeit der Häufigkeit der Bedienfehler und der Bedeutung der Fehler initiiert werden.

Die Ausgabeeinheit ist zur Ausgabe der Meldung ausgelegt. Sie ist eine elektronische Komponente. Die Ausgabeeinheit kann in Software oder Firmware oder Hardware ausgebildet sein. Im ersten Fall kann sie mit der grafischen Benutzeroberfläche (GUI) des Gerätes interagieren, um die Meldung auf der Oberfläche auszugeben.

Die Auswerteeinheit ist zur Auswertung der von der Bedienprüfeinheit erfassten Daten ausgelegt. Sie ist eine elektronische Komponente und kann in Software oder Firmware oder Hardware ausgebildet sein. In den ersten Fällen kann sie als ladbare Applikation bereitgestellt werden. Dabei kann die Funktionalität der Auswerteeinheit vorteilhafterweise auch während des Betriebs angepasst und aktualisiert werden.

Die Sensoreinheit kann mehrere auch unterschiedliche Sensoren umfassen, wie z.B. optische, akustische, thermische Sensoren, Bewegungssensoren, Gyrosensoren, Feuchtigkeitssensoren und/oder Sensoren zur Erfassung von technischen Parametern, wie z.B. Druck oder der Position, Funktion und/oder Stellung von Betriebsmitteln des Gerätes.

Eine weitere Aufgabenlösung besteht in einem Computerprogrammprodukt, das in einen Speicher eines Computers oder eines elektronischen oder medizintechnischen Gerätes geladen oder ladbar ist mit einem Computerprogramm zur Durchführung des oben näher beschriebenen Verfahrens, wenn das Computerprogramm auf dem Computer oder dem elektronischen oder medizintechnischen Gerät ausgeführt wird.

Eine weitere Aufgabenlösung sieht ein Computerprogramm vor zur Durchführung aller Verfahrensschritte des oben näher beschriebenen Verfahrens, wenn das Computerprogramm auf einem Computer, einem elektronischen oder medizintechnischen Gerät ausgeführt wird. Dabei ist es auch möglich, dass das Computerprogramm auf einem für den Computer oder das elektronische oder medizintechnische Gerät lesbaren Medium gespeichert ist.

In der folgenden detaillierten Figurenbeschreibung werden nicht einschränkend zu verstehende Ausführungsbeispiele mit deren Merkmalen und weiteren Vorteilen anhand der Zeichnung besprochen.

### Kurze Beschreibung der Figuren

- Fig. 1: zeigt in einer schematischen Darstellung ein Dialysegerät mit einer Bedienungsprüfeinheit gemäß einer vorteilhaften Ausführungsform der Erfindung.
- Fig. 2: ist ein Beispiel für ein Ablaufdiagramm für ein Betriebsverfahren des Dialysegerätes mit der Bedienungsprüfeinheit.
- Fig. 3: ist eine exemplarische Schema-Darstellung eines Datenaustausches von Signalen und Nachrichten zwischen der Bedienungsprüfeinheit und einer Benutzeroberfläche des Dialysegerätes.
- Fig. 4: zeigt eine detaillierte Darstellung der Bedienungsprüfeinheit beim Betrieb eines Dialysegerätes.

### Detaillierte Beschreibung der Figuren

Im Folgenden wird die Erfindung anhand von Ausführungsbeispielen im Zusammenhang mit den Figuren näher beschrieben.

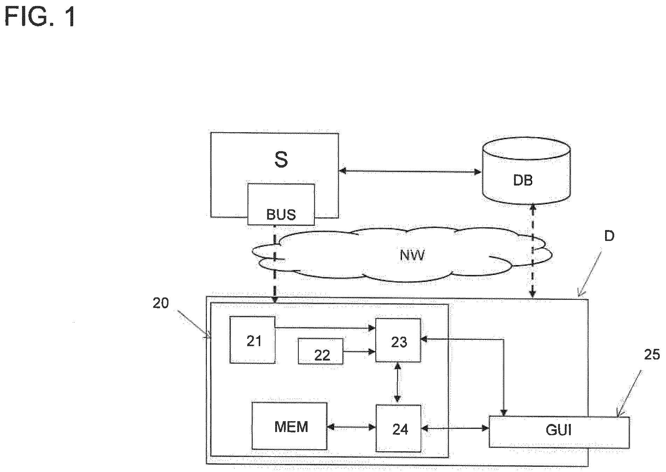

**Fig. 1** zeigt ein Dialysegerät D mit einer Bedienungsprüfeinheit 20, die zur Überprüfung von Bedienaktionen, die am Dialysegerät (im Folgenden auch kurz als Gerät bezeichnet) D ausgeführt werden, auf Korrektheit und zur Erzeugung von anwenderindividueller Bedienhilfen dient. Die Bedienungsprüfeinheit 20 umfasst eine Identifikationseinheit 21, die dazu ausgebildet ist, eine Anwenderidentität des jeweiligen Anwenders zu erfassen und eine Sensoreinheit 22, die ausgebildet ist, eine Bedienaktion des identifizierten Anwenders als IST-Bediendatensatz zu erfassen. Weiterhin umfasst die Bedienungsprüfeinheit 20 eine Verarbeitungseinheit 23, die dazu bestimmt ist, den erfassten IST-Bediendatensatz mit einem in einem Speicher MEM gespeicherten SOLL-Bediendatensatz auf Übereinstimmung zu vergleichen, um bei Abweichung eine anwenderspezifische Meldung zu erzeugen. Die Bedienungsprüfeinheit 20 kann auch eine Auswerteeinheit 24 umfassen, die die dazu ausgebildet ist, auszuwerten, ob, wie oft, in welchem Zeitraum und/oder in welchem Betriebsmodus die Bedienaktion erfasst worden ist und ein Auswertungsergebnis bereitzustellen. Das Auswertungsergebnis kann ebenfalls in dem Speicher MEM lokal gespeichert werden. Weiterhin können die auf der Bedienungsprüfeinheit 20 erfassten oder ermittelten Daten (umfassend das Auswertungsergebnis) auf einer Ausgabeeinheit 25, die als GUI ausgebildet sein kann, ausgebeben werden. Das Gerät D und insbesondere die Verarbeitungseinheit 23 kann mit einem zentralen Server S in Datenaustausch stehen. Dies kann über ein Bussystem BUS und/oder über ein Netzwerk NW erfolgen. Der Server S kann dabei mit dem Dialysegerät D und/oder mit der Bedienungsprüfeinheit 20 in Datenaustausch stehen. Alternativ kann der Server S direkt mit der Verarbeitungseinheit 23 interagieren (in Fig, 1 nicht explizit dargestellt). Der Server S kann mit einer Datenbank DB in Datenaustausch stehen, um z.B. hinterlegte Regeln zum Abgleich zwischen IST-Bediendatensatz und SOLL-Bediendatensatz zu speichern. Zudem kann dort der SOLL-Betriebsdatensatz hinterlegt sein.

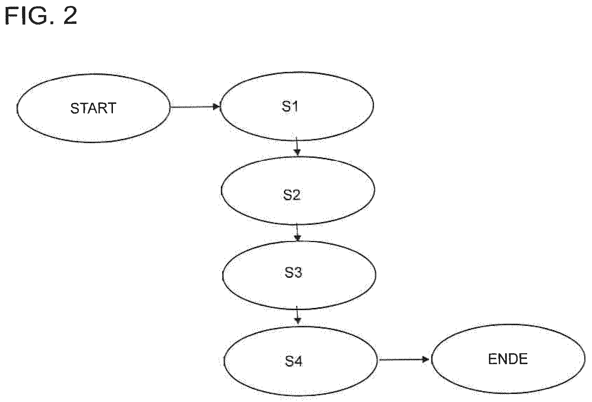

**Fig. 2** zeigt ein Ablaufdiagramm eines Verfahrens gemäß einer bevorzugten Ausführungsform der Erfindung. Nach dem Start wird in Schritt S1 die Anwenderidentität in einem automatischen Detektionsprozess erfasst. Dazu kann auf biometrische Verfahren und/oder auf sonstige Identifikationsprozesse zurückgegriffen werden. Es können auch Identifikationsdaten über eine Schnittstelle eingelesen werden (Anwenderdaten über einen Pager oder über eine Smartcard, wobei unterschiedliche, insbesondere drahtlose Übertragungskanäle zur Anwendung kommen können, wie WLAN, Bluetooth, NFC etc.). In Schritt S2 wird zumindest eine Bedienaktion detektiert; es kann auch eine Folge von Bedienaktionen erfasst werden. Die zumindest eine Bedienaktion des identifizierten Anwenders wird somit in Schritt S2 als IST-Bediendatensatz erfasst. In Schritt S3 wird der erfasste IST-Bediendatensatz mit einem gespeicherten SOLL-Bediendatensatz auf Übereinstimmung verglichen. Abhängig vom Ergebnis des Vergleichs wird ein Prüf- bzw. Vergleichsergebnis bereitgestellt, um bei Abweichung in Schritt S4 eine anwenderspezifische Meldung zu erzeugen. Danach kann das Verfahren enden oder wiederholt angewendet werden.

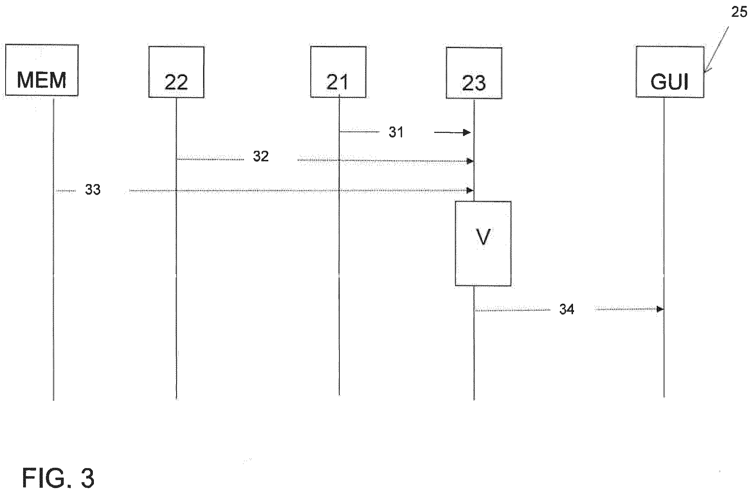

**Fig. 3** zeigt ein Interaktionsdiagramm zwischen den beteiligten elektronischen Modulen. Identitätsdaten 31, die den Anwender eineindeutig identifizieren, werden von der Identifikationseinheit 21 erfasst und an die Verarbeitungseinheit 23 übermittelt. Der erfasste IST-Bediendatensatz 32 wird bei der Betätigung des Bedienelementes BE durch die Sensoreinheit 22 erfasst und ebenfalls an die Verarbeitungseinheit 23 übermittelt. Der SOLL-Bediendatensatz 33 wird aus dem Speicher MEM ausgelesen und an die Verarbeitungseinheit 23 übermittelt. Daraufhin stehen auf der Verarbeitungseinheit 23 alle Daten zur Verfügung, um eine Datenverarbeitung V auszuführen, um ein Prüfergebnis 34 bereitzustellen. Das Prüfergebnis 34 indiziert, ob die erfasste Bedienaktion (der IST-Bediendatensatz 32) der vorkonfigurierten und damit vorhergesehenen, korrekten Bedienung (SOLL-Bediendatensatz 33) entspricht und damit korrekt ist. Das Prüfergebnis 34 kann auf der grafischen Benutzeroberfläche GUI des Gerätes D angezeigt werden. Das Prüfergebnis 34 kann ein Auswerteergebnis umfassen, das noch weitere Aussagen bereitstellt.

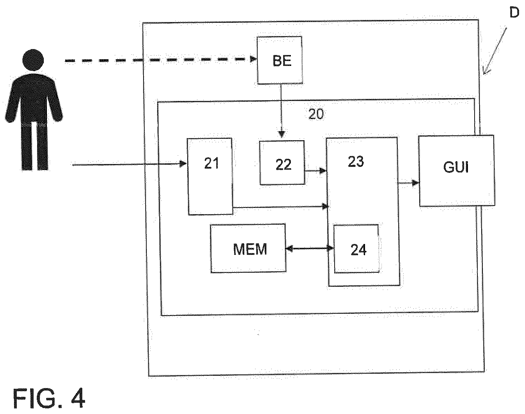

**Fig. 4** zeigt nochmals etwas detaillierter eine Bedienungsprüfeinheit 20, die in ein Dialysegerät D integriert oder an dieses angeschlossen sein kann. In Fig. 4 ist sie in integrierter Form dargestellt. Die Bedienungsprüfeinheit 20 ist somit spezifisch für das jeweilige Dialysegerät D oder für ein anderes medizintechnisches Gerät D, ausgelegt und konfiguriert.

Der Anwender bedient das Dialysegerät D über die notwendigen Bedienaktionen am Gerät, wie z.B. das Öffnen von Abdeckungstüren für das Wechseln eines Filters oder für andere Funktionen. Dazu muss er Bedienelemente BE bzw. Betriebsmittel, Betriebsaggregate und/oder Bedienmittel des Gerätes D bedienen. Die Betätigung des Bedienelementes BE wird automatisch über eine entsprechend angeordnete und ausgelegte Sensoreinheit 22 mit zumindest einem Sensor erfasst. Die Sensoren sind vorteilhafterweise verteilt an unterschiedlichen Betriebsaggregaten angeordnet und umfassen eine Vielzahl von unterschiedlichen Sensortypen (zur Messung einer Position, einer Bewegung eines Bauteils oder zur Messung eines Druckes oder von Strom, Spannung oder anderen technischen Parametern). Die Identität des Anwenders wird ebenso über die entsprechende Identifikationseinheit 21 erfasst. Der von der Identifikationseinheit 21 erfasste Identifikationsdatensatz wird an die Verarbeitungseinheit 23 weitergeleitet. Ebenso werden die Sensordaten, die von der Sensoreinheit 22 erfasst wurden, um eine Bedienaktion des identifizierten Anwenders als IST-Bediendatensatz zu erfassen, an die Verarbeitungseinheit 23 übertragen. Die Verarbeitungseinheit 23 verarbeitet die übermittelten Daten, insbesondere wird der erfasste IST-Bediendatensatz mit dem SOLL-Bediendatensatz auf Übereinstimmung vergleichen, um bei Abweichung die anwenderspezifische Meldung zu erzeugen, die dann auf der GUI des Gerätes D ausgegeben werden kann.

Mit der Einbindung der erfindungsgemäßen Bedienungsprüfeinheit 20 in das medizintechnische System können anwender-spezifische Bedienungseigenarten automatisch erkannt und verarbeitet werden. So können anwenderspezifische Meldungen und Fehlermeldungen auch proaktiv, und somit insbesondere vor der möglicherweise erneut fehlerhaften Bedienung ausgegeben werden und es können Hilfestellungen gegeben werden, um die Fehlersituation erst gar nicht entstehen zu lassen.

Das Gerät kann sich somit auf den Anwender spezifisch "einstellen". Das Gerät kann automatisch anwenderspezifisch konfiguriert werden, indem aufgrund von in zumindest einem zurückliegenden Bedienzeitraum erfassten Bedienaktionen des Anwenders auf zukünftige Bedienaktionen geschlossen wird und Hilfestellungen angeboten werden. Ziel ist eine Fehlervermeidung.

Da jeder Anwender andere Fehler macht, muss die in der Bedienungsprüfeinheit 20 anzuwendende Heuristik anwenderspezifisch sein. Das anwenderspezifische oder anwendertypische Bedienverhalten wird in einer ersten Bedienphase erfasst. Die Ausgabe der berechneten Meldung kann in einer zweiten (geplanten zukünftigen) Bedienphase erfolgen.

Die Erfassung des Bedienverhaltens erfolgt vorzugsweise geräteintern. Die dabei gesammelten Daten werden gespeichert. Die gesammelten Daten (aggregierte IST-Bediendatensätze) werden vorzugsweise lokal (geräteintern) gespeichert und bei jeder Bedienaktion des Anwenders aktualisiert. Zur Speicherung kann insbesondere eine Listenstruktur als Datenstruktur verwendet werden, die im Folgenden beispielhaft widergegeben sein soll:

| **Bedienaktion** | **Anwenderfehler** | **Meldungsnummer** | **Uhrzeit** | **Häufigkeit** |
|---|---|---|---|---|
| TPE | Beim Vorbereiten Filter nicht gedreht | #5312 | 23:12 | 5 |
| CiCa-CVVHDF | Nach Beutelwechsel Filterbeutelklemme geschlossen | #5515 | 9:25 | 10 |
| TPE | Ankoppeltest nicht bestanden | #5315 | 20:55 | 3 |

Die Häufigkeit (letzte Spalte) kann in Bezug zu einem vordefinierbaren Zeitraum berechnet werden. TPE bedeutet dabei Therapeutic Plasma Exchange und CVVHD bezieht sich auf eine kontinuierliche veno-venöse Hämodialyse. Ci-Ca^{®}- Dialysate werden bei Citratantikoagulation verwendet.

Die lokal erfassten Bedienaktionsdaten des Anwenders (die gesammelten IST-Bediendatensätze) können lokal und/oder an anderer Stelle (z.B. zentral) weiterverarbeitet werden, etwa mittels einer statistischen Analyse, um Wahrscheinlichkeiten für typische Behandlungsfehler vorhersagen zu können.

Wenn beispielsweise erfasst wird, dass die erfassten Meldungen sich über den Verlauf der Zeit ändern (indem z.B. bestimmte Fehler nicht mehr gemacht werden), dann wird diese Änderung ebenfalls automatisch erfasst und zur Gerätesteuerung umgesetzt. So kann es im Set-Up des Gerätes konfiguriert werden, dass Meldungen dann nicht mehr ausgegeben und angezeigt werden, wenn sie über einen vorkonfigurierbaren Zeitraum der Fehlerfreiheit nicht mehr erfasst worden sind (z.B. nicht mehr bei den letzten 5 oder 10 Bedienungen oder in einem Zeitraum von 1 Tag oder 1 Woche).

Wie in der beispielhaften Tabelle oben gezeigt, kann eine Uhrzeit für die Bedienaktion miterfasst werden. Wenn die gesammelten Daten z.B. indizieren, dass ein bestimmter Fehler (im Beispiel TPE) immer nur nachts und nicht tagsüber gemacht wird (oder in bestimmten Zeiträumen), dann kann das Gerät automatisch so angesteuert werden, dass die Meldung zur Fehlervermeidung nur nachts und nicht tagsüber (nur in den bestimmten Zeiträumen) ausgegeben wird.

Die Meldung kann auch einen Hinweis auf die Fehlerursache und deren Auswirkung(en) umfassen.

Für die Meldungen wird vorzugsweise eine feste, vordefinierbare Struktur verwendet. Dies dient dazu, die Meldungen von anderen Gerätemeldungen unterscheidbar zu machen, um die Gerätebedienung effizienter und einfacher auszugestalten. Die Meldungen können z.B. ein spezifisches Erscheinungsbild (look and feel) haben.

Abschließend sei darauf hingewiesen, dass die Beschreibung der Erfindung und die Ausführungsbeispiele grundsätzlich nicht einschränkend in Hinblick auf eine bestimmte physikalische Realisierung der Erfindung zu verstehen sind. Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren. Es liegt somit z.B. ebenso im Rahmen der Erfindung alternativ oder kumulativ zur grafischen Benutzeroberfläche 25 andere Bedienoder Steuerelemente des medizinischen Gerätes zur Ausgabe des Verarbeitungsergebnisses 34 bereitzustellen. Dabei kann es sich z.B. auch um externe Ausgabegeräte handeln. Für einen Fachmann ist es insbesondere offensichtlich, dass die Erfindung nicht nur für Dialysegeräte angewendet werden kann, sondern auch für andere medizintechnische Geräte D, bei denen eine korrekte Bedienung überprüft werden muss. Falls keine korrekte Bedienung bestimmt wurde, soll ein Verarbeitungsergebnis 34 erzeugt und gegebenenfalls ausgegeben werden.

Darüber hinaus können die Bauteile des medizinischen Systems zur Überwachung von Bedienaktionen und der Bedienungsprüfeinheit 20 auf mehrere physikalische Produkte verteilt realisiert sein.

Der Schutzbereich der vorliegenden Erfindung ist durch die Ansprüche gegeben und wird durch die in der Beschreibung erläuterten oder den Figuren gezeigten Merkmale nicht beschränkt.

### BEZUGSZEICHEN

- D: Medizintechnisches Gerät, insbesondere Dialysegerät
- 20: Bedienungsprüfeinheit
- 21: Identifikationseinheit
- 22: Sensoreinheit
- 23: Verarbeitungseinheit
- 24: Auswerteeinheit
- 25: Grafische Benutzeroberfläche GUI

- 31: Identitätsdaten
- 32: IST-Bediendatensatz
- 33: SOLL-Bediendatensatz
- 34: Prüfergebnis

- S1: Erfassen einer Anwenderidentität
- S2: Erfassen zumindest einer Bedienaktion
- S3: Vergleich IST-Bediendatensatz mit SOLL-Bediendatensatz
- S4: Erzeugen und ggf. Ausgeben eines Prüfergebnisses

## Patentansprüche

1. Bedienungsprüfeinheit (20) für ein Dialysegerät mit einer Identifikationseinheit (21), die dazu ausgebildet ist, eine Anwenderidentität eines Anwenders zu erfassen, wobei die Bedienungsprüfeinheit umfasst:
- eine Sensoreinheit (22), die ausgebildet ist, in einer ersten Betriebsphase eine Bedienaktion des identifizierten Anwenders als IST-Bediendatensatz zu erfassen, wobei die Bedienaktion in eine Abfolge von aufeinander folgenden, vordefinierten aufeinander abgestimmten Bedienaktionen eingebettet ist;
- eine Verarbeitungseinheit (23), die dazu bestimmt ist, den erfassten IST-Bediendatensatz mit einem in einem Speicher (MEM) gespeicherten SOLL-Bediendatensatz auf Übereinstimmung zu vergleichen, um bei Abweichung eine anwenderspezifische Meldung zu erzeugen;
- wobei die Bedienungsprüfeinheit (20) eine Ausgabeeinheit (25) umfasst oder mit dieser in Datenaustausch steht, die dazu ausgebildet ist, die erzeugte anwenderspezifische Meldung, den erfassten IST-Bediendatensatz, den SOLL-Bediendatensatz und/oder ein Auswerteergebnis auszugeben, wobei die erzeugte anwenderspezifische Meldung in einer zweiten Betriebsphase ausgegeben wird bevor die Bedienaktion ausgeführt wird, wenn in einer vorhergehenden Betriebsphase des identifizierten Anwenders für die jeweilige Bedienaktion eine anwenderspezifische Meldung erzeugt worden ist
**dadurch gekennzeichnet, dass**
die erzeugte anwenderspezifische Meldung aufgrund von vorhergehenden Fehlerüberwachungen von Bedienaktionen für dieselbe Prozedur, die eine Abfolge von Bedienaktionen umfasst, und für den identischen Anwender festgestellt worden ist, dass dem Anwender hier Fehler unterlaufen sind.

2. Bedienungsprüfeinheit (20) nach Patentanspruch 1, **dadurch gekennzeichnet, dass** die Bedienungsprüfeinheit (20) eine Auswerteeinheit (24) umfasst, die dazu ausgebildet ist, auszuwerten, ob, wie oft, in welchem Zeitraum und/oder in welchem Betriebsmodus die Bedienaktion erfasst worden ist und ein Auswertungsergebnis bereitzustellen.

3. Dialysegerät, mit einer Bedienungsprüfeinheit (20) nach einem der vorangehenden Patentansprüche.

4. Verfahren zum anwenderspezifischen Überwachen von Bedienaktionen beim Betrieb eines Dialysegerätes, **dadurch gekennzeichnet, dass** während eines Betriebs des Dialysegerätes zunächst eine Anwenderidentität und daraufhin in einer ersten Betriebsphase eine Bedienaktion des identifizierten Anwenders als IST-Bediendatensatz erfasst wird (S1, S2), wobei die Bedienaktion in eine Abfolge von aufeinander folgenden, vordefinierten aufeinander abgestimmten Bedienaktionen eingebettet ist und, dass der erfasste IST-Bediendatensatz mit einem gespeicherten SOLL-Bediendatensatz auf Übereinstimmung verglichen wird (S3), um bei Abweichung eine anwenderspezifische Meldung zu erzeugen (S4), wobei die erzeugte anwenderspezifische Meldung, der erfasste IST-Bediendatensatz, der SOLL-Bediendatensatz und/oder ein Auswerteergebnis ausgegeben werden, wobei die erzeugte anwenderspezifische Meldung in einer zweiten Betriebsphase ausgegeben wird bevor die Bedienaktion ausführt wird, wenn in einer vorhergehenden Betriebsphase des identifizierten Anwenders für die jeweilige Bedienaktion eine anwenderspezifische Meldung erzeugt worden ist und damit aufgrund von vorhergehenden Fehlerüberwachungen von Bedienaktionen für dieselbe Prozedur, die eine Abfolge von Bedienaktionen umfasst, und für den identischen Anwender festgestellt worden ist, dass dem Anwender hier Fehler unterlaufen sind.

5. Verfahren nach dem vorangehenden Verfahrensanspruch, **dadurch gekennzeichnet, dass** mit dem Erfassen der Bedienaktion weitere Metadaten, insbesondere ein Zeitstempel, erfasst werden.

6. Verfahren nach einem der vorangehenden Verfahrensansprüche, **dadurch gekennzeichnet, dass** die Anwenderidentität erfasst wird (S1), indem ein Identifikationsprozess ausgeführt, der auf einem optischen Verfahren und/oder einem biometrischen Verfahren und/oder auf einer Eingabe von Schlüsseldaten und/oder durch Einlesen von Daten eines mobilen Datenträgers basiert.

7. Verfahren nach einem der vorangehenden Verfahrensansprüche, **dadurch gekennzeichnet, dass** die anwenderspezifische Meldung auf einem Monitor (25) des Dialysegerätes auf einem Touchscreen-Monitor, ausgegeben wird.

8. Verfahren nach einem der vorangehenden Verfahrensansprüche, **dadurch gekennzeichnet, dass** die anwenderspezifische Meldung eine Quittierung seitens des Anwenders erfordert und eine fehlerhafte Bedienung indiziert und/oder Bedienhinweise umfasst.

9. Verfahren nach einem der vorangehenden Verfahrensansprüche, **dadurch gekennzeichnet, dass** ein Ausgeben der anwenderspezifischen Meldung aktivierbar und de-aktivierbar ist und/oder automatisch nach Eintreten eines vordefinierbaren Ereignisses oder eines vordefinierbaren Zeitraums aktiviert bzw. deaktiviert wird.

10. Verfahren nach einem der vorangehenden Verfahrensansprüche, **dadurch gekennzeichnet, dass** alle erfassten Bedienaktionen von allen Anwendern aggregiert und für jeden Anwender aufgeschlüsselt gespeichert und/oder ausgegeben werden.

11. Verfahren nach dem unmittelbar vorangehenden Verfahrensanspruch, **dadurch gekennzeichnet, dass** der SOLL- Bediendatensatz in Abhängigkeit von der erfassten Anwenderidentität und/oder von einem Betriebsmodus anwenderindividuell konfiguriert wird.

12. Computerprogramm zur Durchführung aller Verfahrensschritte des vorstehend beanspruchten Verfahrens, wenn das Computerprogramm auf einem Computer oder auf einem Dialysegerät ausgeführt wird.

## Claims

1. Operation verification unit (20) for a dialysis device with an identification unit (21) designed to record the user identity of a user, wherein the operation verification unit comprises:
- a sensor unit (22) designed to detect, in a first operating phase, an operating action of the identified user as an ACTUAL operating data record, wherein the operating action is embedded in a sequence of successive, predefined, coordinated operating actions;
- a processing unit (23) designed to compare the recorded ACTUAL operating data record with a TARGET operating data record stored in a memory (MEM) in order to generate a user-specific message in the event of a deviation;
- wherein the operation verification unit (20) comprises an output unit (25) or is in data exchange with the latter, which is designed to output the generated user-specific message, the recorded ACTUAL operation data record, the TARGET operation data record and/or an evaluation result, wherein the generated user-specific message is output in a second operating phase before the operating action is executed if a user-specific message has been generated for the respective operating action in a previous operating phase of the identified user
**characterized in that**
the generated user-specific message has been determined on the basis of previous error monitoring of operating actions for the same procedure, which comprises a sequence of operating actions, and for the identical user, that the user has made errors here.

2. Operation verification unit (20) according to claim 1, **characterized in that** operation verification unit (20) comprises an evaluation unit (24) which is designed to evaluate whether, how often, in what period of time and/or in what operating mode the operating action was recorded and to provide an evaluation result.

3. Dialysis device with an operation verification unit (20) according to one of the preceding patent claims.

4. Method for user-specific monitoring of operating actions during operation of a dialysis device, **characterized in that** during operation of the dialysis device, a user identity is first recorded and then, in a first operating phase, an operating action of the identified user is recorded as an ACTUAL operating data record (S1, S2), wherein the operating action is embedded in a sequence of successive, predefined, coordinated operating actions, and that the recorded ACTUAL operating data record is compared with a stored TARGET operating data record for consistency (S3) in order to generate a user-specific message in the event of a deviation (S4), wherein the generated user-specific message, the recorded ACTUAL operating data record, the TARGET operating data record and/or an evaluation result are output, wherein the generated user-specific message is output in a second operating phase before the operating action is executed, if a user-specific message has been generated for the respective operating action in a previous operating phase of the identified user and it has thus been determined on the basis of previous error monitoring of operating actions for the same procedure, which comprises a sequence of operating actions, and for the identical user that the user has made errors here.

5. Method according to the preceding method claim, **characterized in that** further metadata, in particular a time stamp, is recorded when the operating action is recorded.

6. Method according to one of the preceding method claims, **characterized in that** the user identity is recorded (S1) by performing an identification process based on an optical method and/or a biometric method and/or the entry of key data and/or by reading data from a mobile data carrier.

7. Method according to one of the preceding method claims, **characterized in that** the user-specific message is output on a monitor (25) of the dialysis device on a touchscreen monitor.

8. Method according to one of the preceding method claims, **characterized in that** the user-specific message requires acknowledgment by the user and indicates incorrect operation and/or includes operating instructions.

9. Method according to one of the preceding method claims, **characterized in that** outputting the user-specific message can be activated and deactivated and/or is automatically activated or deactivated after a predefined event or a predefined period of time has occurred.

10. Method according to one of the preceding method claims, **characterized in that** all recorded operating actions by all users are aggregated and stored and/or output broken down for each user.

11. Method according to the immediately preceding method claim, **characterized in that** the target operating data record is configured individually for each user depending on the recorded user identity and/or an operating mode.

12. Computer program for performing all steps of the method claimed above when the computer program is executed on a computer or on a dialysis device.

## Revendications

1. Unité de contrôle de fonctionnement (20) pour un appareil de dialyse avec une unité d'identification (21) qui est conçue pour enregistrer l'identité d'un utilisateur, l'unité de contrôle de fonctionnement comprenant :
- une unité de capteur (22) conçue pour enregistrer, dans une première phase de fonctionnement, une action de commande de l'utilisateur identifié sous forme d'un ensemble de données de commande RÉELLES, l'action de commande étant intégrée dans une séquence d'actions de commande successives, prédéfinies et coordonnées entre elles ;
- une unité de traitement (23) qui est destinée à comparer l'enregistrement de données de commande RÉEL enregistré avec un enregistrement de données de commande THÉORIQUE stocké dans une mémoire (MEM) afin de générer un message spécifique à l'utilisateur en cas d'écart ;
- l'unité de contrôle de commande (20) comprenant une unité de sortie (25) ou étant en communication avec celle-ci, qui est conçue pour émettre le message spécifique à l'utilisateur généré, l'enregistrement de données de commande RÉEL enregistré, l'enregistrement de données de commande THÉORIQUE et/ou un résultat d'évaluation, le message spécifique à l'utilisateur généré étant émis dans une deuxième phase de fonctionnement avant que l'action de commande ne soit exécutée, si un message spécifique à l'utilisateur a été généré pour l'action de commande correspondante dans une phase de fonctionnement précédente de l'utilisateur identifié
**caractérisé en ce que**
le message spécifique à l'utilisateur généré a été déterminé sur la base de contrôles d'erreurs précédents d'actions de commande pour la même procédure, qui comprend une séquence d'actions de commande, et pour le même utilisateur, que l'utilisateur a commis des erreurs ici.

2. Unité de contrôle de commande (20) selon la revendication 1, **caractérisée en ce que** l'unité de contrôle de commande (20) comprend une unité d'évaluation (24) qui est conçue pour évaluer si, à quelle fréquence, dans quel intervalle de temps et/ou dans quel mode de fonctionnement l'action de commande a été enregistrée et pour fournir un résultat d'évaluation.

3. Appareil de dialyse, avec une unité de contrôle de commande (20) selon l'une des revendications précédentes.

4. Procédé de surveillance spécifique à l'utilisateur des actions de commande lors du fonctionnement d'un appareil de dialyse, **caractérisé en ce que,** pendant le fonctionnement de l'appareil de dialyse, une identité d'utilisateur est d'abord enregistrée, puis, dans une première phase de fonctionnement, une action de commande de l'utilisateur identifié est enregistrée sous forme d'enregistrement de données de commande RÉEL (S1, S2), l'action de commande étant intégrée dans une séquence d'actions de commande successives, prédéfinies et coordonnées et **en ce que** l'enregistrement de données de commande RÉEL enregistré est comparé à un enregistrement de données de commande THÉORIQUE mémorisé afin de vérifier la concordance (S3) et, en cas de divergence, de générer un message spécifique à l'utilisateur (S4), le message spécifique à l'utilisateur généré, l'enregistrement des données de commande réelles saisies, l'enregistrement des données de commande théoriques et/ou un résultat d'évaluation étant alors émis, le message spécifique à l'utilisateur généré étant émis dans une deuxième phase de fonctionnement avant que l'action de commande ne soit exécutée, si, dans une phase de fonctionnement précédente de l'utilisateur identifié, un message spécifique à l'utilisateur a été généré pour l'action de commande respective et qu'il a ainsi été constaté, sur la base de contrôles d'erreurs précédents d'actions de commande pour la même procédure, qui comprend une séquence d'actions de commande, et pour le même utilisateur, que l'utilisateur a commis des erreurs ici.

5. Procédé selon la revendication de procédé précédente, **caractérisé en ce que,** lors de la saisie de l'action de commande, d'autres métadonnées, en particulier un horodatage, sont saisies.

6. Procédé selon l'une des revendications de procédé précédentes, **caractérisé en ce que** l'identité de l'utilisateur est enregistrée (S1) en exécutant un processus d'identification basé sur un procédé optique et/ou un procédé biométrique et/ou sur la saisie de données clés et/ou par la lecture de données d'un support de données mobile.

7. Procédé selon l'une des revendications précédentes, **caractérisé en ce que** le message spécifique à l'utilisateur est affiché sur un écran (25) de l'appareil de dialyse, sur un écran tactile.

8. Procédé selon l'une des revendications de procédé précédentes, **caractérisé en ce que** le message spécifique à l'utilisateur nécessite une confirmation de la part de l'utilisateur et indique une erreur de manipulation et/ou comprend des instructions d'utilisation .

9. Procédé selon l'une des revendications précédentes, **caractérisé en ce que** l'émission du message spécifique à l'utilisateur peut être activée et désactivée et/ou est automatiquement activée ou désactivée après la survenue d'un événement prédéfinissable ou d'une période prédéfinissable.

10. Procédé selon l'une des revendications de procédé précédentes, **caractérisé en ce que** toutes les actions de commande enregistrées par tous les utilisateurs sont agrégées et stockées et/ou émises de manière ventilée pour chaque utilisateur.

11. Procédé selon la revendication de procédé immédiatement précédente, **caractérisé en ce que** l'ensemble de données de commande de consigne est configuré individuellement pour chaque utilisateur en fonction de l'identité de l'utilisateur enregistrée et/ou d'un mode de fonctionnement.

12. Programme informatique pour exécuter toutes les étapes du procédé revendiqué cidessus, lorsque le programme informatique est exécuté sur un ordinateur ou sur un appareil de dialyse.
